# EUROPEAN PATENT SPECIFICATION

(11) **EP 3 094 278 B1**
(45) Date of publication and mention of the grant of the patent: **25.11.2020**
(21) Application number: 14809116.8
(22) Date of filing: 03.11.2014
(51) Int. Cl.: A61B 90/00, A61L 2/26, A61B 50/30, A61B 50/36

(54) **BOX FOR PACKAGING AND/OR DISPOSAL OF INSTRUMENTS AND ITS USE**
SCHACHTEL ZUM VERPACKEN UND/ODER ENTSORGEN VON INSTRUMENTEN UND DEREN VERWENDUNG
BOÎTE POUR EMBALLER ET/OU JETER DES INSTRUMENTS, ET SON UTILISATION

(30) Priority: 14.01.2014 PT 10740114
(43) Date of publication of application: 23.11.2016
(73) Proprietor: Bastos Viegas, S.A., 4560-164 Guilhufe - Penafiel (PT)
(72) Inventor: VIEGAS NIETO GUIMARÃES, Luis Salvador, 4405-847 Vilar do Paraíso (PT); CORREIA SOARES RIBEIRO, Raquel, 4580-402 Gondalães - Paredes (PT)
(74) Representative: Ferreira, Maria Silvina
(86) International application number: PCT/IB2014/065769
(87) International publication number: WO 2015/107400

(56) References cited:
- WO-A1-96/25966
- WO-A1-03/094999
- DE-U1- 9 312 355
- US-A- 5 372 787
- US-A- 6 012 577

## Description

### Technical domain

The present application describes a box for packaging and/or disposal of instruments and its use.

### Background

To meet the essential requirements of the medical devices directive, sterile packages must follow all requirements defined in the appropriate harmonized standards, according to which materials and systems designed for sterile barrier must be packaged to ensure the required protection to preserve performance characteristics during transport and storage. Throughout the present application it is understood that "sterile barrier system" describes the minimum package required to perform the required functions in medical package: allow sterilization, provide an acceptable microbial barrier and allow aseptic presentation.

One key point of the harmonized package standard for sterile medical devices are performance tests which evaluate interaction between the product and the package system after application of stresses and pressures from production process, sterilization, distribution, storage and environmental aspects, in order to ensure preservation of integrity of the sterile barrier system. The package qualification test is conducted after exposure to the specified sterilization process and in an extreme situation regarded as the worst case for the system. Some of these test methods include compression, vibration and shock tests of the package, with the article under investigation being in the interior to demonstrate compliance with the same standard.

Another relevant point also described in the standard refers that the sharp articles included in the sterile systems must be protected in order to guarantee that the user, the sterile barrier system and the device itself will not suffer injury or damage. The same standard also foresees a possibility for the inclusion of associated components in the sterile barrier system such as inner wraps, instrument organizer trays, tray liners and containment devices around the medical device, in order to facilitate organization, drying or aseptic presentation.

These associated components must present several requirements, namely: be non-toxic and compatible with the devices to be packaged as well as with the sterile barrier system; provide protection to the device and system during storage and transport until its use; allow and be compatible with the sterilization process and be able to withstand the conditions of that same process; do not undergo chemical or physical changes that adversely affect performance and the safety of the medical device incorporated therein; do not compromise aseptic presentation; ideally allow easy identification of contents; be stored in controlled environment to maintain cleanliness and suitability for use.

At hospital level reusable instruments are generally packaged, sterilized and stored on hard metal containers, preferably made of aluminum or a polymeric material. These containers are used to maintain sterility of the medical devices until use, in accordance with the requirements established by the standards in practice. Due to its structural characteristics and performance, the containers act as a reusable sterile barrier. Such solution cannot be applied for the industrial package of disposables articles due to high associated costs.

At industrial level, the most common sterile barrier systems for disposable articles are for example: sterilization pouches or sleeves; polyamide/polypropylene polyamide/polypropylene/polyethylene or polyethylene packaging, and paper or nonwoven polyethylene fibers; polyethylene film bags with window in nonwoven polyethylene fibers. All these systems comprise a rigid, semi-rigid or flexible film and medical grade paper or nonwoven polyethylene fibers which allow passage of the sterilizing agent. Other existing package systems consist exclusively of films; nevertheless, these are only suitable for sterilization by irradiation. Selection of the package system depends on the sterilization method and product features. Cost associated with this package will be higher the greater the thickness and robustness of the raw materials and package systems and higher the substrate specificity that allows the sterilization of devices. The sterilization pouches or sleeves sometimes used in a hospital environment for individual reprocessing of small and lightweight instruments are not viable at industrial level. The raw materials comprising these package systems are easily punctured by the sharp ends of the instruments, especially while packaging these packages in cardboard boxes and during transport. The incidence of these perforations will be higher the lower the resistance and higher the free space inside the package which allows for greater movement of the instrument within.

In alternative to the increase in resistance properties and features in the primary package, that are inefficient and/or costly, imply the protection of instruments or its sharp surfaces thereby preventing direct contact with the package. Placement of instruments in the disposable rigid plastic open trays and posteriorly involved in wrapping fields or sterilization sheets also does not prove to be sufficiently effective and perforation of the substrates and later, of the package, may occur.

Alternatively, the protection of each cutting or perforating surface through systems or protective cases is a costly solution due to the size and configurations being conditioned by the instrument design, and when individually applied to each article, adding significant costs due to skilled labor. In addition, the same configuration aspect and number of instruments is also a limiting criterion while choosing placement in card bases or other rigid material enclosing it.

US5031768 shows a tray for placing and sorting instruments after sterilization or cleaning, which may be disposed at the end of the procedure. Thus, the sterile instruments are removed from the sterile barrier system and orderly placed in the tray in accordance with the procedure to which they are intended. The closure of the tray with the instruments therein prevents external dust contamination during storage until the time of use, nevertheless the preservation of sterility is not an objective of the invention. The article as described in US5031768 and the one described in the present application have in common the possibility of disposal at the end of the procedure. However, both present distinct functions. The first constitutes a system for organizing and storing instruments after sterilization and prior to use without preserving the sterility of the content. The second is a system that promotes the integrity of the sterile barrier system until the moment of use of the devices in order to maintain the sterility of articles therein WO 96/25966 A1 discloses a box for packaging and/or disposal of instruments made in a single piece comprising two long side surfaces and two short side surfaces extending from a bottom surface to the top of the box wherein the bottom surface comprises at least a raised surface placed parallel to the two short side surfaces.

Given the growing concern regarding handling, inspection and disposal of contaminated material, several solutions have emerged in the market such as boxes, pads, receptacles, containers, bearers and other systems to place devices such as needles, sutures, syringes, scalpels, blades and other sharp devices, that allow a recount of the material used in the procedure as well as safe disposal. These types of safety devices are increasingly requested by health related services and professionals and are even inserted as additional components in the compositions of the sets. The box now presented may also be used as a disposal receptacle with the advantage of being dimensioned to the actual needs of the service or procedures, unlike other systems that are exclusively designed for disposal of contaminated material.

### Summary

The present application describes a box for packaging and/or disposal of instruments which comprises:
- a bottom surface that comprises at least one raised surface parallel to the short side walls;
- side surfaces;
- right flap of the upper surface;
- left flap of the upper surface;
- spring system or fitting;
- at least one taper pin perpendicular to the bottom surface.

In one embodiment, the side surfaces of the box for packaging and/or disposal of instruments comprises two long side surfaces and two short side surfaces.

In one embodiment, the right and left flaps of the upper surface of the box for packaging and/or disposal of instruments present openings.

In another embodiment, openings in the box for packaging and/or disposal of instruments are located in the central area of the upper surface.

In yet another embodiment, the box for packaging and/or disposal of instruments comprises a recessed frieze in the inner surface of each flap.

In one embodiment, the contact surface of the left flap of the box for packaging and/or disposal of instruments is placed at a lower level than the contact surface of the right flap.

In one embodiment, the box for the packaging and/or disposal of instruments comprises at least one receptacle therein.

In another embodiment, the edges of the box for packaging and/or disposal of instruments are rounded.

In yet another embodiment, the packaging box and/or disposal of instruments shows at least one adhesive on the outer part of the bottom surface.

In one embodiment, the final closure system of the box for packaging and/or disposal of instruments comprise one adhesive surface that adheres to the flaps, covering the openings that facilitate the sterilization process and allow closing the box with the contaminated devices in its interior at the end of the procedure.

In another embodiment, the adhesive and the adhesive surface that adheres to the flaps of the box for packaging and/or disposal of instruments are a single element.

In yet another embodiment, the box for packaging and/or disposal of instruments is made by metallic material and/or composite material and/or polymeric material.

In one embodiment, the box for packaging and/or disposal of instruments is made in polypropylene.

The present application further describes the use of the box for packaging and/or disposal of instruments in the packaging and/or transport in the sterile barrier system and/or disposal of disposable instruments. The invention is defined by claim 1 and the use of the box as disclosed in claim 10. Further embodiments of the invention are defined by the dependent claims.

### General description

The present application describes a box (10) for the packaging and/or disposal of instruments, to be placed inside the sterile barrier system, designed for use at industrial level for packaging single devices and sets. In the present application, it is considered that sets refer mostly to devices packaged in a single package and sterilized together and which are intended to be used in a specific medical-surgical procedure. The box (10) prevents possible perforations from the instruments in the sterilization package during its packaging, sterilization, storage and transport.

The main objective of the box (10) is to ensure integrity of the sterile barrier system until further use in medical services. Thus, the box (10) now presented is not by itself a sterile barrier system, since it does not complies with requirements such as preservation of sterility after exit from sterilizer, but represents a protection complement to this system so that it complies with the applicable requirements. Being a disposable article, the box (10) also works as a suitable device for the disposal of these instruments and other sharp articles after use.

The box (10) disclosed in this application can be fabricated with rigid material, such as metal, composite or polymer, even more preferably in polypropylene, in which disposable instruments are placed, for example instruments used in medical or surgical procedures. The box (10) may be made by rigid material, such as polypropylene, since this is a low cost and widely used material in medical, domiciliary, emergency and operating services in disposable articles like plastic instruments, bowls, trays, receptacles, spatulas, among others. On the other hand, it was also considered in the design the need for effective protection of the sterile barrier system that would not posteriorly resulted in an increase in the time spent or difficulty for preparing the material to be used by health professionals.

The box (10) with the instruments therein is placed in the sterile barrier system thereby avoiding direct contact of the instruments with the referred package and consequently its perforation, being so unnecessary to resort to more complex package systems. To avoid any damage in the sterile barrier system resulting from the box (10) itself no edge or blunt surface is present. Moreover this is the first stage of the process that is performed at industrial level, which briefly comprises the following steps:
- Instruments and other articles that may be included in the box (10) are grouped together and placed in its interior;
- Box (10) is closed, for example by a spring system, fitting or other;
- Additionally, an adhesive (27) may be applied;
- The box (10) is placed inside the sterile barrier system, which is then sealed;
- The sterile barrier system, is packaged in transport boxes;
- Sterilization;
- Storage and transport.

The adhesive (27) that can be further placed in the box (10) will make it possible for health professionals to secure the box (10) to the work surface at the beginning of the procedure and will also allow closing the box (10) with contaminated devices in its interior at the end of the procedure, for posterior disposal.

To ease the sterilization process, box (10) comprises openings (16) on its surfaces. The sterilizing agent passing through the sterile barrier system may thus have immediate access to the components placed inside. Since several instruments may present long and thin edges, the size and location of these openings (16) in the box (10), preferably in a central position of the upper surface, minimizes the risk of passing from one of the ends to the exterior. Preferably, the box (10) must comprise the same number of openings (16) on each flap so that a balance in the mold during injection is possible and the arrangement must also be such that the final closure adhesive (27) may reach them and proceed to its total closure.

Box (10) comprises at least one bottom surface (11). On the other hand, when used by health professionals, the spring system or fitting that joins together the flaps keeps the instruments inside the box (10), ensuring the instrument's permanence inside during sterilization, packaging, storage and transport procedures. This spring system or fitting may be operated with one finger of the user, thereby enabling its opening and providing a simple and fast access to the interior. This feature is particularly important in the design of the article since, in most cases, the user will be wearing gloves and, since these are sterile articles, they should have the lowest possible manipulation.

Given the need to develop a box (10) made in a material that ensures effective protection from possible perforations and suitable for use in a wide range of sizes and different configurations of instruments, the need to have sufficient size to accommodate the greater number of instruments was taken into consideration, but which, on the other hand, could also be adapted to a small number of instruments while allowing the inclusion of other devices such as receptacles for liquids, wound dressing and surgical drapes materials, thereby preventing empty spaces and consequently increased costs in terms of package, sterilization, storage and transport.

Additionally, the box (10) may have at least one adhesive system (27) applied presenting two distinct functions for different phases of the process. The end of the adhesive (27) that is applied to the bottom surface (11) of the box (10) allows, when detached, the box (10) to be secured to the worktable at the beginning of the procedure, thus preventing accidental movements of the box (10) with the sterile instruments inside and consequently possible contamination. On the other hand at the end of the procedure, already with the instruments inside the box (10), it is possible to detach the other edge of the same adhesive (27) that is partially applied on one of the flaps and secure it on the two flaps and on the passage openings of the sterilizing agent. Like this, the box (10) is closed with additional security and may be discarded, thus avoiding any accidental contact between the contaminated instruments and the health professionals or the officials responsible for transport and cleaning services and also avoiding possible perforation in the bags intended for disposal. Another alternative to this adhesive system (27) may be the inclusion of a second piece allowing the closure of the passage openings of the sterilizing agent.

To allow inspection and recount of the articles used after closure, the box (10) might be transparent or might present at least one transparent surface or part of a transparent surface.

### Description of figures

For easier understanding of the technique annex figures are joined, which represent preferred embodiments which, however, are not intended to limit the subject matter of this application.

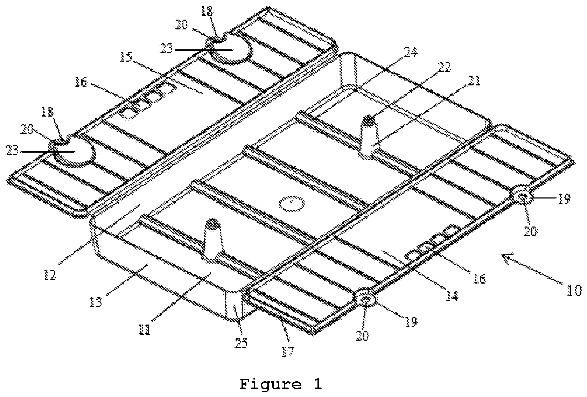

**Figure 1** illustrates schematic representation of the box for packaging and/or disposal of instruments in open position, the following reference numbers representing:
   10 - box;
   11 - bottom surface;
   12 - long side surface;
   13 - short side surface;
   14 - right flap of the upper surface;
   15 - left flap of the upper surface;
   16 - openings for ease of the sterilization process;
   17 - recessed frieze;
   18 - left flap contact surface;
   19 - right flap contact surface;
   20 - central opening of the spring system or fitting;
   21 - support taper pin;
   22 - fitting surface of the support pin;
   23 - cavity;
   24 - raised surface;
   25 - box edge.
**Figure 2** illustrates a schematic representation of the box for packaging and/or disposal of instruments in open position, the following reference numbers representing:
   10 - box;
   11 - bottom surface;
   12 - long side surface;
   13 - short side surface;
   14 - right flap of the upper surface;
   15 - left flap of the upper surface.
**Figure 3** illustrates schematic representation of the box for packaging and/or disposal of instruments in closed position, the following reference numbers representing:
   10 - box;
   12 - long side surface;
   13 - short side surface;
   14 - right flap of the upper surface;
   15 - left flap of the upper surface;
   16 - openings to ease the sterilization process;
   19 - right flap contact surface;
   22 - fitting surface of the support pin;
   23 - cavity;
   25 - box edge.

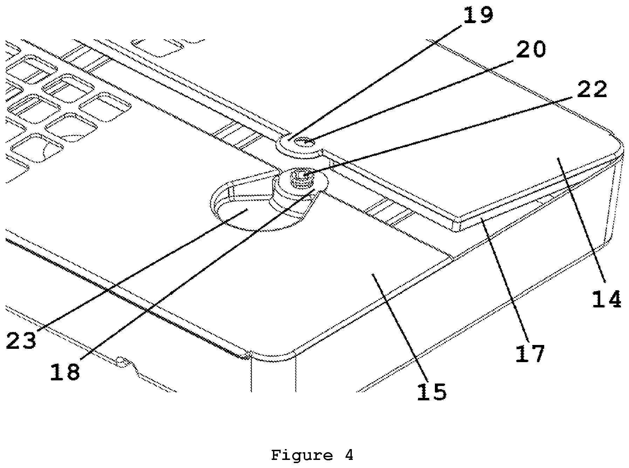

**Figure 4** illustrates schematic representation of the spring system or fitting of the box for packaging and/or disposal of instruments, the following reference numbers representing:
   14 - right flap of the upper surface;
   15 - left flap of the upper surface;
   17 - recessed frieze;
   18 - left flap contact surface;
   19 - right flap contact surface;
   20 - central opening of the spring system or fitting;
   22 - fitting surface of the support pin;
   23 - cavity.

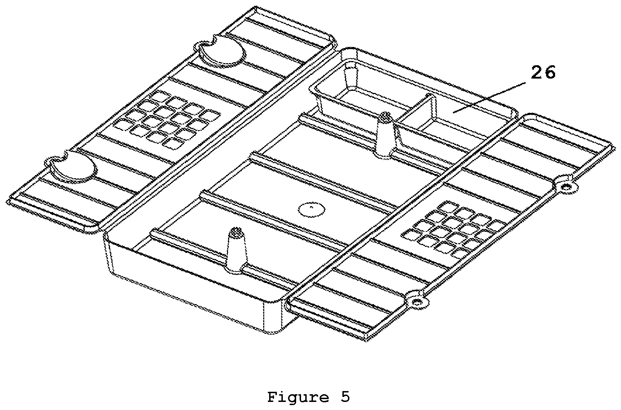

**Figure 5** illustrates schematic representation of the receptacle for small amounts of liquids of the box for packaging and/or disposal of instruments in open position, the following reference number representing:
   26 - receptacle for liquid.

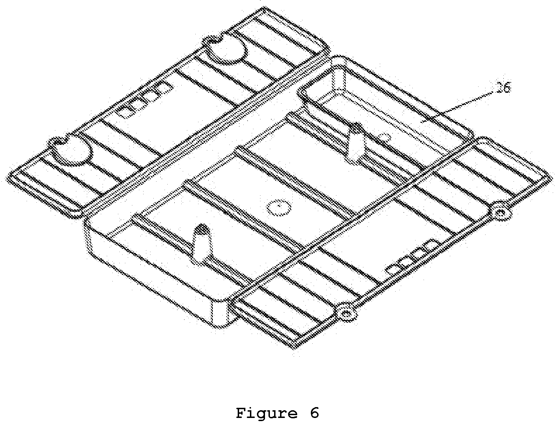

**Figure 6** illustrates schematic representation of the receptacle for liquid of the box for packaging and/or disposal of instruments in open position, the following reference number representing:
   26 - receptacle for liquid.

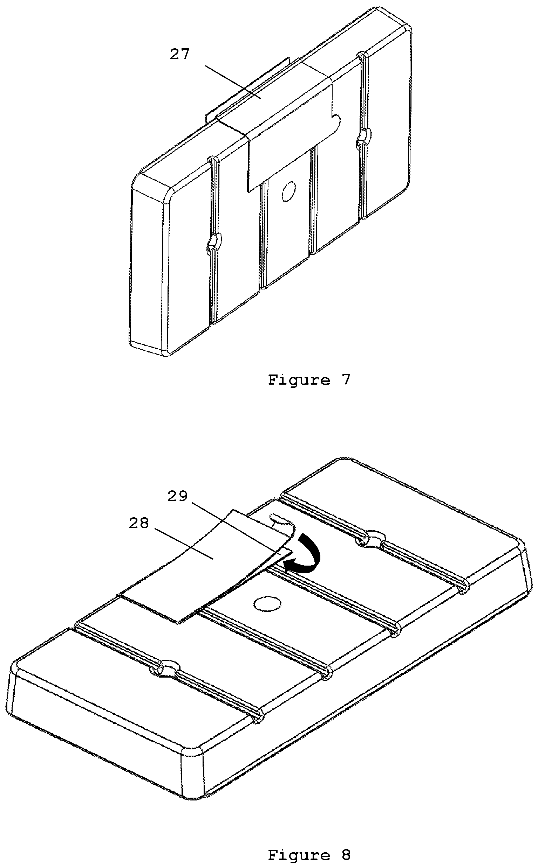

**Figure 7** illustrates schematic representation of a view of the adhesive applied to the box for packaging and/or disposal of instruments in open position, where the following reference number represent:
   27 - adhesive.
**Figure 8** illustrates schematic representation of a bottom view of the box with one area of adhesive used for securing the box to the worktable showed in detail, the following reference numbers representing:
   28 - removable surface for detaching the adhesive area to secure to the worktable;
   29 - adhesive area to secure to the worktable.

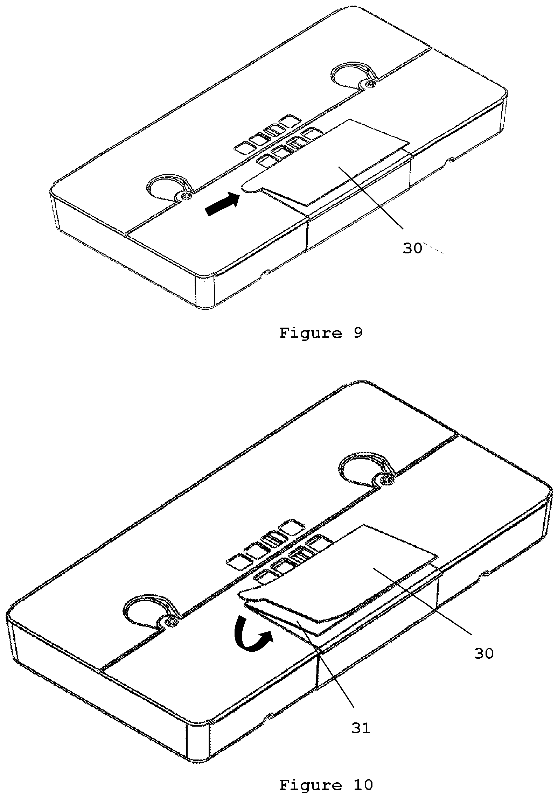

**Figure 9** illustrates schematic representation of a top view of the box with one adhesive area used for the final closure of the box and safe disposal showed in detail, the following reference number representing:
   30 - removable surface for detaching the adhesive area to be secured on the flaps and on the openings that ease the sterilization process.
**Figure 10** illustrates schematic representation of a view of the box with one adhesive area (27) used for the final closure of the box and safe disposal showed in detail, the following reference number representing:
   30 - removable surface for detaching the adhesive area to be secured on the flaps of the box and on the openings that ease the sterilization process;
   31- adhesive area to be fixed over the flaps of the box and over the openings that facilitate the sterilization process.

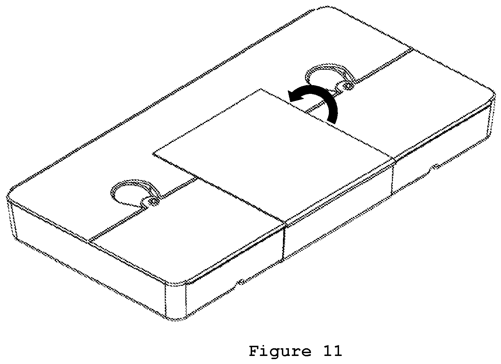

**Figure 11** illustrates schematic representation of a view of the box with the adhesive applied for safe disposal.

### Description of embodiments

The present application describes a box (10) for packaging and/or disposal of instruments prior to their inclusion in the sterile barrier system, made in a rigid material, as for example metal, composite or polymer, even more preferably in polypropylene, and structured in such a way as to prevent the accidental exit of instruments from its interior during packaging, sterilization, storage and transport.

The box (10) presents non-blunt surfaces and edges so as to prevent any damage to the sterile barrier system and openings in the surfaces that ease the sterilization process. Also included is a drawing in relief on the inner side of the bottom surface (11) in which instruments are placed. To avoid instruments to be completely leaned against this bottom surface (11), which would hinder its reach by health professionals; the referred surface is irregular with some elevated areas. Distance between the most elevated areas, preferably comprise up to 15 cm, more preferably up to 11 cm, enables the instruments to rest on them, thus facilitating its reach through the lower areas.

Along this bottom surface (11) taper pins may be placed, perpendicular to the referred surface, and which extend up to the top of the box (10). These pins serve as support to the spring system or fittings, and also prevent further movement of the instruments. The number and location of these pins are features in the box (10) designed not only to restrict the number or type of possible instruments to include in its interior but also to ensure the versatility of including other components such as receptacles for liquids, fields or dressing material. The number and location of the pins obeys the following factors:
- The pins confer rigidity to the flaps, to the spring system or fitting and limit the movement of the instruments. However, this number cannot significantly reduce the total capacity of the box (10);
- The central location of the pins located allows the two flaps to have a very rough area making viable the required balance of the mold;
- The distance between the two pins is between 5 and 15 cm, preferably 10 cm which allows the placement of the most common sizes and packs of folded fields;
- The distance between the pins and the side surfaces (12, 13) of the box (10) is sufficient to place the receptacle (26) for liquids.

Box (10) allows incorporating a large number of instruments or, alternatively, if such is not justified, instruments in combination with other devices.

On the exterior side of the bottom surface (11) part of an adhesive (27) is applied which after being detached allows securing the box (10) to the work area before its opening thus avoiding possible accidental movements that may result in the fall and contamination of the material.

The procedure being finished the instruments used, now contaminated, can be placed again inside the box (10). To ensure safe closure of the box (10), the adhesive (27) extends to one of its flaps, being partially applied on it. This adhesive (27) is then detached in its entirety and applied over both the flaps and over the openings that facilitate the sterilization process. This way, the devices used as well as fluids, such as blood, are retained in the box (10).

The material for production of the box (10) can be transparent to allow visual inspection and counting of the articles after its closure. This system can then be detached from the worktable and safely discarded in appropriate containers.

### Examples of application

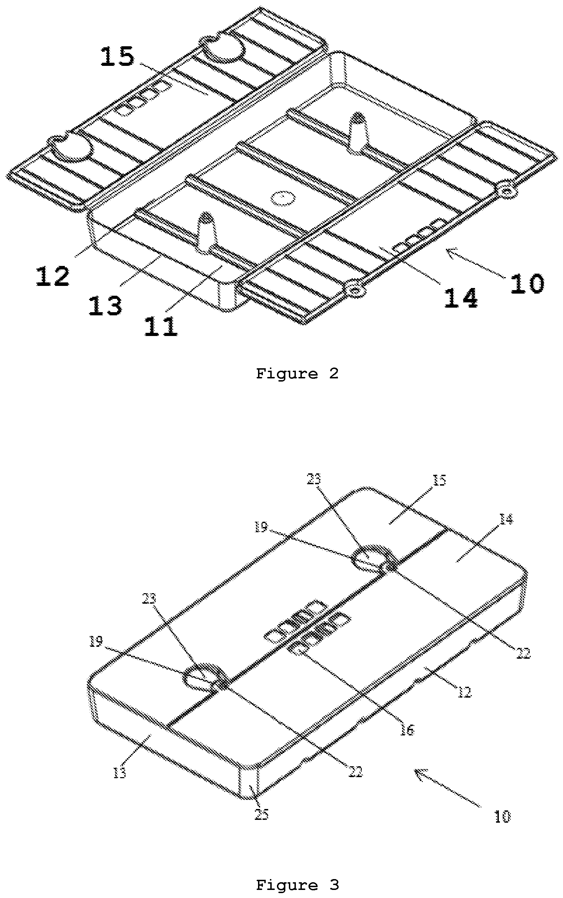

Figures 1 and 3 present two possible embodiments of the present invention, generally designated as box (10), in open and close positions, respectively.

The box (10) is provided with a single, preferably rectangular piece, comprised by a bottom surface (11), two long side surfaces (12) and two short side surfaces (13) extending from the bottom surface (11) to the top of the box (10).

The two long side surfaces (12) extend parallel to the bottom surface (11) until they meet in the middle of the box (10) thereby forming two flaps (14 and 15) that comprise the upper surface of the box (10). These flaps (14 and 15) have openings (16) that facilitate the passage of the sterilizing agent into the container during the sterilization process. These openings are located and dimensioned to minimize the risk of any end of an instrument of passing, regardless its shape or size.

On the interior surface of each flap (14 and 15), along its ends a recessed frieze is placed (17) at a lower level that fits in the side surfaces of the box (12 and 13) and prevents the output of thinner instruments after its closure. The frieze and the spring system or fitting of the box (10) are shown in detail in figure 4.

Both flaps (14 and 15) have contact surfaces (18 and 19) between themselves which delimit a central opening (20). The contact surface of the left flap (18) is placed at a lower level than the contact surface of the right flap (19) so that these surfaces and respective openings are superimposable. Thus, when the box (10) is closed, the contact surface of the right flap (19) is superimposed on the contact surface of the left flap (18) and maintain a central opening (20) common to both through which the closure fitting of the system is performed.

The spring system or fitting of the box (10) is made between the surface of the support taper pin (22) and the central openings of the contact surfaces of the flaps (20). The support taper pins (21) are arranged at the bottom surface (11) of the box (10) and extend vertically above the contact surfaces of the flaps. These support pins (21) in addition to confer opening and closure of the box (10), reduce the displacement of the instruments and other components placed inside. The fitting surface of each support pin (22) is divided into four equal parts that engage the central opening of the contact surfaces of the flaps (20) by manual pressure. On the right flap of the upper surface (14), near the contact surface, there is a cavity (23) designed to allow easier access to the contact surface of the right flap (19) so that, through manual pressure the flap may be released from the spring system or fitting and consequently the flap may be opened.

In order to ease the removal of instruments placed inside the box (10), the bottom surface (11) of the box (10) has some raised surfaces (24) placed parallel to the short side walls and spaced between themselves in order to contemplate different sizes of instruments. The edges of the box (25) are rounded to avoid disruptions in the surrounding fields and/or package. To ensure greater versatility the possibility of including other components in its interior was also considered and as such a receptacle (26) for small quantities of liquids as disinfectants was also designed. This receptacle (26), represented in figures 5 and 6, is a separate piece that might be attached to the box (10) occupying one of the surfaces between the locking pin and the nearest short side wall. This way the remaining area of the box (10) stays free for the inclusion of other articles. This point has also been considered in the location of the locking pins so that the distance between them would be sufficient as to allow placement of the most common sizes of various wound dressing materials such as compresses and surgical drapes.

In figures 7 and 8 is possible to verify the outer part of the bottom surface (11) of the box (10) which will be in contact with the worktable. To ensure greater stability to the box (10) avoiding accidental displacements along the work surface, part of an adhesive (27) is applied on this surface which, by removal of the protective base (28) may be detached and glued to the worktable. The adhesive surface will be detached at the end of the procedure after the final closure of the box (10).

In order to be able to convert this box (10) for packaging and/or disposal in an appropriate device for the disposal of sharp articles or contaminated articles after use, the adhesive (27) extends until one of the flaps of the box (10) onto which is partly applied as represented in Figures 9 and 10. The adhesive area (31) is applied over the flaps to cover the openings that promote sterilization, through the removal of the protective surface (30). This way, in addition of providing a safe closure system to the box (10), the adhesive (27) also covers the referred openings that could act as a means for contaminants passage from the interior to the exterior.

The present embodiment is not, naturally, in no way restricted to the embodiments described herein and a person of ordinary skill in the area may provide many possibilities for modifications thereof without moving away from the invention, as defined in the claims.

The preferred embodiments described above are obviously combined with each other. The following claims additionally define preferred embodiments.

## Claims

1. Box for packaging and/or disposal of instruments made in a single piece comprising:
- two long side surfaces (12) and two short side surfaces (13) extending from a bottom surface (11) to the top of the box (10);
- the bottom surface (11) comprises at least a raised surface (24) placed parallel to the two short side surfaces (13) **characterised by**
- a right flap (14) comprising a contact surface (19) delimiting a central opening (20) and a left flap (15) comprising a contact surface (18) also delimiting a central opening (20), adjacent to the two long side surfaces (12), the flaps (14,15) forming the top of the box (10) when closed, wherein the flaps (14,15) have openings (16) located in the central zone of the right flap (14) and the left flap (15) and adapted to facilitate the passage of a sterilizing agent into the container during a sterilization process;
- at least one support taper pin (21) having a support pin fitting surface (22), wherein the support taper pin (21) is placed along and perpendicular to the bottom surface (11), and wherein the at least one support taper pin (21) is adapted to support a spring system or fittings and to reduce the displacement of the instruments and other components placed inside box (10);
- a spring system or fitting made between the surface of a support taper pin (22) and central openings of the contact surfaces of flaps (20) in contact surface (18) and contact surface (19),
wherein the fitting surface of each support pin (22) is divided into four equal parts that engage the central opening of the contact surfaces of the flaps (20) by manual pressure wherein the spring system or fitting is adapted to allow the opening and closing of box (10);
- a recessed frieze (17) in the inner surface of each flap (14,15) that fits in the two long side surfaces (12) and the two short side surfaces (13).

2. Box for packaging and/or disposal of instruments according to claim 1, wherein the contact surface (18) of the left flap (15) is placed at a lower level than the contact surface (19) of the right flap (14).

3. Box for packaging and/or disposal of instruments according to any one of the previous claims, wherein it comprises at least one receptacle (26) in its interior.

4. Box for packaging and/or disposal of instruments according to any one of the previous claims, wherein its edges are rounded.

5. Box for packaging and/or disposal of instruments according to any one of the previous claims, wherein it presents at least one adhesive (27) on the outer part of the bottom surface (11).

6. Box for packaging and/or disposal of instruments according to any one of the previous claims, wherein the adhesive (27) comprises an adhesive surface that adheres to the flaps (14,15), covering the openings (16).

7. Box for packaging and/or disposal of instruments according to any of the claims 5 and 6, wherein the adhesive (27) and the adhesive surface that adheres to the flaps (14,15) are a single element.

8. Box for packaging and/or disposal of instruments according to any one of the previous claims, wherein it is made in metallic material and/or composite material and/or polymeric material.

9. Box for packaging and/or disposal of instruments according to the previous claim, wherein it is made in polypropylene.

10. Use of the box for packaging and/or disposal of instruments described in any of the previous claims in the packaging and/or transport and/or disposal of disposable instruments in the sterile barrier system.

## Patentansprüche

1. Aus einem Teil hergestelltes Gehäuse zur Verpackung und/oder Entsorgung von Instrumenten bestehend aus:
Zwei langen Seitenflächen (12) und zwei kurzen Seitenflächen (13), die sich von der Bodenfläche (11) bis zur Oberseite des Gehäuses (10) erstrecken;
die Bodenfläche (11) weist mindestens eine erhabene Oberfläche (24) auf, die parallel zu den zwei kurzen Seitenflächen (13) verläuft, **gekennzeichnet durch**
eine rechte Klappe (14), die eine Kontaktfläche (19) enthält, welche eine zentrale Öffnung (20) umschließt und eine
linke Klappe (15) die eine Kontaktfläche (18) enthält, welche ebenfalls eine zentrale Öffnung (20) umschließt und sich
nahe der zwei langen Seitenflächen (12) befindet, wobei die Klappen (14,15) im geschlossenen Zustand die Oberseite des Gehäuses (10) bilden und worin die Klappen (14/15)
Öffnungen (16) aufweisen, die sich im mittleren Bereich der rechten Klappe (14) und der linken Klappe (15) befinden und geeignet sind, den Durchfluss eines Sterilisationsmittels in den Behälter während des Sterilisationsprozesses zu erleichtern;
mindestens einen stützenden Kegelbolzen (21), der eine Passfläche (22) aufweist, wobei sich der stützende Kegelbolzen (21) längs der und senkrecht zur Bodenfläche (11) befindet und worin der mindestens eine stützende Kegelbolzen (21) geeignet ist, ein Federsystem oder Armaturen aufzunehmen und die Verschiebung der innerhalb des Gehäuses (10) befindlichen Instrumente und anderer Komponenten zu verringern;
ein Federsystem oder eine Armatur
zwischen der Auflagefläche des stützenden Kegelbolzens (22) und den zentralen Öffnungen der Kontaktflächen der Klappen (20) in der Kontaktfläche (18) und Kontaktfläche (19),
worin die Passfläche jedes Stützbolzens (22) in vier gleiche Teile geteilt ist, welche in die zentrale Öffnung der Kontaktflächen der Klappen (20) durch manuellen Druck einrasten und worin das Federsystem oder die Armatur geeignet ist, das Öffnen oder Schließen des Gehäuses (10) zu ermöglichen;
ein ausgesparter Fries (17) in der Innenfläche jeder Klappe (14,15), der in die zwei langen Seitenflächen (12) und zwei kurzen Seitenflächen (13) hineinpasst.

2. Gehäuse zur Verpackung und/oder Entsorgung von Instrumenten gemäß Anspruch 1, worin die Kontaktfläche (18) der linken Klappe (15) auf einer niedrigeren Ebene als die Kontaktfläche (19) der rechten Klappe (14) liegt.

3. Gehäuse zur Verpackung und/oder Entsorgung von Instrumenten gemäß einem der vorherigen Ansprüche, worin dieses mindestens einen Behälter (26) im Innenbereich aufweist.

4. Gehäuse zur Verpackung und/oder Entsorgung von Instrumenten gemäß einem der vorherigen Ansprüche, worin dessen Kanten abgerundet sind.

5. Gehäuse zur Verpackung und/oder Entsorgung von Instrumenten gemäß einem der vorherigen Ansprüche, worin dieses mindestens einen Aufkleber (27) am äußeren Teil der Bodenfläche (11) aufweist.

6. Gehäuse zur Verpackung und/oder Entsorgung von Instrumenten gemäß einem der vorherigen Ansprüche, worin der Aufkleber (27) eine Klebefläche aufweist, die an den Klappen (14,15) haftet und die Öffnungen (16) abdeckt.

7. Gehäuse zur Verpackung und/oder Entsorgung von Instrumenten gemäß einem der Ansprüche 5 und 6, worin der Aufkleber (27) und die an den Klappen (15,15) haftende Klebefläche ein Einzelelement darstellen.

8. Gehäuse zur Verpackung und/oder Entsorgung von Instrumenten gemäß einem der vorherigen Ansprüche, worin dieses aus Metallwerkstoff und/oder aus Verbundmaterial und/oder aus Polymerwerkstoff hergestellt wird.

9. Gehäuse zur Verpackung und/oder Entsorgung von Instrumenten gemäß dem vorherigen Anspruch, worin dieses aus Polypropylen hergestellt wird.

10. Verwendung des in einem der vorherigen Ansprüche beschriebenen Gehäuses zur Verpackung und/oder Entsorgung von Instrumenten bei der Verpackung und/oder dem Transport und/oder der Entsorgung von Einweginstrumenten im Sterilbarrieresystem.

## Revendications

1. Boîte pour l'emballage et/ ou l'élimination d'instruments faite d'une seule pièce comprenant :
deux surfaces latérales longues (12) et deux surfaces latérales courtes (13) s'étendant à partir d'une surface inférieure (11) jusqu'au sommet de la boîte (10) ;
la surface inférieure (11) comprend au moins une surface surélevée (24) placée parallèlement aux deux surfaces latérales courtes (13) **caractérisée par**
un rabat droit (14) comprenant une surface de contact (19) délimitant une ouverture centrale (20) et un
un rabat gauche (15) comprenant une surface de contact (18) délimitant également une ouverture centrale (20), adjacente
aux deux surfaces latérales longues (12), les rabats (14, 15) formant le haut de la boîte (10) lorsqu'ils sont fermés, dans laquelle les rabats (14, 15)
ont des ouvertures (16) situées dans la zone centrale du rabat droit (14) et du rabat gauche (15) et adaptées pour faciliter le passage d'un agent stérilisant dans le récipient durant un processus de stérilisation ;
au moins une goupille conique de support (21) ayant une surface d'ajustement de goupille de support (22), dans laquelle la goupille conique de support (21) est placée le long et perpendiculaire à la surface inférieure (11), et dans laquelle l'au moins une goupille conique de support (21) est adaptée pour supporter un système de ressort ou des ajustements et pour réduire le déplacement des instruments et autres éléments placés à l'intérieur de la boîte (10) ;
un système de ressort ou un ajustement réalisé
entre la surface d'une goupille conique de support (22) et les ouvertures centrales des surfaces de contact des rabats (20) dans la surface de contact (18) et la surface de contact (19),
dans laquelle la surface d'ajustement de chaque goupille de support (22) est divisée en quatre parties égales qui engagent l'ouverture centrale des surfaces de contact des rabats (20) par pression manuelle dans laquelle le système de ressort ou l'ajustement est adapté pour permettre l'ouverture et la fermeture de la boîte (10) ;
une frise en creux (17) dans la surface intérieure de chaque rabat (14, 15) qui s'adapte dans les deux surfaces latérales longues (12) et les deux surfaces latérales courtes (13).

2. Boîte pour l'emballage et/ ou l'élimination d'instruments selon la revendication 1, dans laquelle la surface de contact (18) du rabat gauche (15) est placée à un niveau inférieur par rapport à la surface de contact (19) du rabat droit (14) .

3. Boîte pour l'emballage et/ ou l'élimination d'instruments selon l'une quelconque des revendications précédentes, dans laquelle celle-ci comprend au moins un réceptacle (26) dans son intérieur.

4. Boîte pour l'emballage et/ ou l'élimination d'instruments selon l'une quelconque des revendications précédentes, dans laquelle ses bords sont arrondis.

5. Boîte pour l'emballage et/ ou l'élimination d'instruments selon l'une quelconque des revendications précédentes, dans laquelle celle-ci présente au moins un adhésif (27) sur la partie extérieure de la surface inférieure (11).

6. Boîte pour l'emballage et/ ou l'élimination d'instruments selon l'une quelconque des revendications précédentes, dans laquelle l'adhésif (27) comprend une surface adhésive qui adhère aux rabats (14, 15), en couvrant les ouvertures (16).

7. Boîte pour l'emballage et/ ou l'élimination d'instruments selon l'une quelconque des revendications 5 et 6, dans laquelle l'adhésif (27) et la surface adhésive qui adhère aux rabats (14, 15) sont un seul élément.

8. Boîte pour l'emballage et/ ou l'élimination d'instruments selon l'une quelconque des revendications précédentes, dans laquelle celle-ci est faite d'un matériau métallique et/ ou d'un matériau composite et/ ou d'un matériau polymère.

9. Boîte pour l'emballage et/ ou l'élimination d'instruments selon les revendications précédentes, dans laquelle celle-ci est faite de polypropylène.

10. Utilisation de la boîte pour l'emballage et/ ou l'élimination d'instruments décrite dans l'une quelconque des revendications précédentes dans l'emballage et/ ou le transport et/ ou l'élimination d'instruments jetables dans le système de barrière stérile.
